# NOUVEAU FASCICULE DE BREVET EUROPEEN

(11) **EP 2 168 991 B2**
(45) Date de publication et mention de la décision concernant l'opposition: **01.09.2021**
(45) Mention de la délivrance du brevet: 09.10.2013
(21) Numéro de dépôt: 09177977.7
(22) Date de dépôt: 20.05.2005
(51) Int. Cl.: C08F 220/26, C08F 220/06, C08F 220/38, A61K 8/81, A61Q 5/02, A61Q 5/06

(54) **Nouveaux copolymères éthyléniques, compositions les comprenant et procédé de traitement**
Neue Ethylencopolymere, diese enthaltende Zusammensetzungen und Behandlungsverfahren
New ethylene copolymeres, compositions comprising the same and treatment method

(30) Priorité: 13.07.2004 FR 0451512
(43) Date de publication de la demande: 31.03.2010
(62) Demande divisionnaire de: 05291089.0
(73) Titulaire: L'Oréal, 75008 Paris (FR)
(72) Inventeur: Mougin, Nathalie, 75011, PARIS (FR); Jegou, Gwenaëlle, 91240, Saint Michel sur Orge (FR)
(74) Mandataire: L'Oreal

(56) Documents cités:
- EP-A1- 0 003 235
- EP-A1- 0 995 791
- EP-A2- 0 324 568
- WO-A-02/44267
- DE-U1- 20 103 544
- FR-A1- 2 912 648

## Description

La présente invention a trait à de nouveaux polymères, à leur utilisation notamment en cosmétique, ainsi qu'aux compositions les comprenant.

Il est connu d'employer des polymères dans le domaine cosmétique, et notamment en capillaire, par exemple pour apporter de la tenue ou du coiffant à la chevelure. De nombreuses compositions cosmétiques, et en particulier les compositions capillaires dites de "hair styling", qui se présentent sous la forme d'aérosols ("sprays") de gels, de mousses ou de shampooings, contiennent des résines ou des polymères. Ainsi, le document US-A-6 193 961 de ISP décrit un terpolymère homogène de N-vinyllactame de préférence de N-vinylpyrrolidone ou de N-vinylcaprolactame, d'acrylate de diméthylaminoalkyle ou de diméthylaminoakylacrylamide et d'un monomère polysiloxane. Le document US-A-5 502 136 concerne un procédé de préparation de copolymères de vinylpyrrolidone et d'acétate de vinyle par polymérisation radicalaire. Toutefois, on a constaté que ces polymères ne présentent pas une élimination suffisante au shampooing.

Dans le domaine des compositions capillaires dites "non rincées", telles que les produits de coiffage de type laques, gel ou spray coiffant, on est constamment à la recherche de bons polymères coiffants. Ainsi, dans WO200209656, il est décrit des polymères hydrophobes à base d'acrylate de butyle, apportant un effet coiffant repositionnable. Dans EP1201223, on décrit des copolymères à base de (méth)acrylate d'alkyles permettant de coiffer et recoiffer la chevelure à volonté. Toutefois, dans ces deux cas, la cosmétique au toucher est insuffisante.

Ainsi, il n'est pas connu de polymères apportant du coiffant avec une cosmétique acceptable et une élimination au shampoing aisée.

La présente invention a pour but de proposer des polymères capables d'apporter un réel effet coiffant tout en conservant une cosmétique acceptable aux compositions, et en particulier une bonne élimination au shampoing.

Après de nombreuses recherches, la demanderesse a mis en évidence que l'utilisation de polymères comprenant entre autre des monomères du type (méth)acrylate de polyéthylèneglycol tels que définis ci-après, pouvait permettre la réalisation de compositions coiffantes ayant une cosmétique adéquate.

Des polymères contenant des motifs (méth)acrylate de polyéthylène glycol (MPEG) sont décrits dans l'art antérieur.

Ainsi, il est connu par EP372546, des copolymères à base de MPEG et de monomères de type (méth)acrylamides d'alkyles en C1-C8, pouvant comprendre des monomères cationiques. Ces polymères cationiques ne s'éliminent toutefois pas aisément au rinçage. Par ailleurs, ils ne permettent pas de générer des effets cosmétiques adéquats, notamment un dépôt sur le cheveu qui soit suffisant pour apporter les propriétés recherchées.

Le document JP2002-322219 décrit des polymères contenant des motifs MPEG en association avec des monomères hydrophobes à base de polypropylène glycol (PPO) ou de polyoxyde de tétraméthylène, et des monomères cationiques. Ces polymères comprennent donc des monomères hydrophobes et on a constaté qu'ils ne permettent pas d'obtenir des propriétés cosmétiques satisfaisantes.

Il est également connu par le brevet JP2002-284627 une composition comprenant des polymères cationiques dans lequel des monomères de type PEG sont associés à des monomères comprenant des motifs amines quaternaires. Or, la présence de motifs quaternaires peut induire, au fur et à mesure des applications, un surcroît de dépôt qui peut conduire, dans certains cas, à une mauvaise cosmétique et engendrer une difficulté d'élimination au shampooing.

On connaît encore par JP2003-055164 des polymères contenant des motifs du type MPEG; toutefois ces polymères sont réticulés, ce qui rend difficile le contrôle de leur synthèse, ainsi que leur élimination au shampoing.

Le document JP2000-302649 décrit une composition capillaire comprenant un polymère qui comprend des monomères cationiques ou amphotères, des monomères à groupement polyéther, notamment de type MPEG ou PPO, ainsi que des monomères optionnels qui peuvent être principalement hydrophobes (méthacrylate de stéaryle).

Il est également connu par le brevet JP07-285831, des compositions capillaires comprenant un polymère qui comprend des monomères de type MPEG en association avec des monomères ioniques, cationiques ou amphotères, et des monomères additionnels de type (méth)acrylates d'alkyle en C1-C24, principalement hydrophobes.

Toutefois, la présence de monomères hydrophobe, de type acrylate de butyle ou de stéaryle, ne permet pas d'obtenir des propriétés cosmétiques adéquates, notamment une bonne cosmétique et une élimination au shampoing adéquates.

On connaît encore la demande WO03/075867 qui décrit des copolymères blocs linéaires comportant une séquence poly(alkylène glycol) encadrée par deux séquences éthyléniques. Ces polymères présentent le défaut d'avoir une séquence centrale de type poly(alkylène glycol) de masse élevée qui confère au polymère une forte cristallinité, ce qui peut conduire à des produits opaques et/ou présentant un caractère gras.

Par ailleurs, dans l'ensemble des polymères décrits dans ces documents, les monomères ioniques sont principalement des monomères cationiques, qui peuvent former un dépôt sur le cheveu lorsqu'ils sont mis en présence de tensioactif anionique. Or, dans certains cas, par exemple dans le cas des produits de coiffage non rincé de type laques, il peut être intéressant de disposer de compositions pouvant être éliminées facilement notamment au lavage, par exemple avec un shampooing comprenant un tensioactif anionique.

Il revient à la demanderesse d'avoir constaté qu'il était préférable d'utiliser des polymères comprenant des MPEG et des motifs anioniques pour obtenir un dépôt satisfaisant de polymère sur le cheveu.

La demanderesse a mis en évidence de nouveaux polymères permettant d'apporter un effet coiffant et conditionneur aux produits cosmétiques capillaires, tout en étant facilement éliminable par exemple au shampooing.

De manière surprenante, les polymères selon l'invention possèdent des propriétés cosmétiques intéressantes, par exemple lors de l'application dans une formulation de type laque ou shampooing. On a en effet constaté que les cheveux se démêlent facilement lors du shampooing, et qu'ils présentent après application, de la douceur; après séchage, les compositions selon l'invention permettent également une remise en forme de la chevelure particulièrement intéressante.

En outre, l'application de ces compositions permet d'obtenir un effet coiffant facilement éliminable au shampoing.

Enfin, les polymères selon l'invention sont aisément formulables, par exemple en flacon-pompe.

Sans être tenu par la présente explication, on peut penser que ces propriétés peuvent notamment être dues à la présence de motifs (méth)acrylate de PEG (MPEG) au sein de la chaîne de polymère, motifs qui contribuent en grande part à l'effet obtenu.

Un objet de la présente invention est donc un copolymère éthylénique comprenant, en % en poids par rapport au poids total du polymère, :
- a) 10-80% en poids d'un ou plusieurs monomères de formule (I) telle que définie ci-après;
- b) 20-90% en poids d'au moins un monomère anionique choisi parmi l'anhydride maléique et les monomères de formule (II) telle que définie ci-après; et
- c) 0,1 à 35% en poids d'au moins un monomère hydrophile non ionique de formule (III) telle que définie ci-après.

Un autre objet de l'invention est une composition comprenant, dans un milieu physiologiquement acceptable, au moins un tel copolymère.

La présente invention a pour avantage de proposer des polymères généralement véhiculables dans l'eau, c'est-à-dire solubles ou dispersibles dans l'eau, ce qui permet de les employer de manière avantageuse dans des compostions cosmétiques, notamment de soin de la peau, ou capillaires, généralement à base aqueuse.

Par hydrosoluble ou soluble dans l'eau, on entend que le polymère forme une solution limpide dans l'eau, à raison d'au moins 5% en poids, à 25°C.

Par hydrodispersible ou dispersible dans l'eau, on entend que le polymère forme dans l'eau, à une concentration de 5% en poids, à 25°C, une suspension ou dispersion stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.

Dans la suite de la présente description, on entendra par 'radical cyclique' un radical monocyclique ou polycyclique, qui peut se présenter lui-même sous forme d'un ou plusieurs cycles, saturés et/ou insaturés, éventuellement substitués (par exemple cyclohexyle, cyclodécyle, benzyle ou fluorényle), mais également un radical qui comprend un ou plusieurs desdits cycles (par exemple p-tertbutylcyclohexyle ou 4-hydroxybenzyle).

On entendra par'radical saturé et/ou insaturé', les radicaux totalement saturés, les radicaux totalement insaturés, y compris aromatiques, ainsi que les radicaux comportant une ou plusieurs doubles et/ou triples liaisons, le reste des liaisons étant des liaisons simples.

Le copolymère éthylénique selon l'invention comprend donc au moins un monomère de formule (I), qui peut être présent seul ou en mélange, : 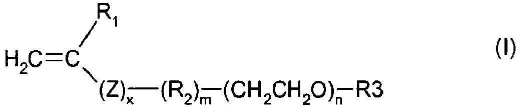 dans laquelle :
- R1 est un atome d'hydrogène ou un radical méthyl;
- Z est un groupement divalent -COO-;
- x est 1;
- m est 0
- n est un entier compris entre 3 et 300 inclus;
- R3 est un atome d'hydrogène ou un radical méthyle;

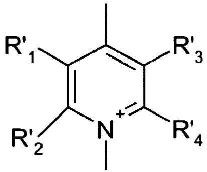

De préférence, n est compris entre 5 et 200 inclus, et encore mieux entre 7 et 100 inclus, voire entre 9 et 50 inclus.

Parmi les monomères de formule (I) particulièrement préférés, on peut citer :
- le (méth)acrylate de poly(éthylène glycol) dans lequel R3 = H;
- le (méth)acrylate de méthyl-poly(éthylène glycol), aussi appelé (méth)acrylate de méthoxypoly(éthylèneglycol), dans lequel R3 = méthyle;

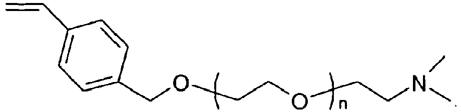

Des exemples de monomères commerciaux sont :
- le CD 350 (méthacrylate de méthoxy-poly(éthylène glycol 350) et le CD 550 (méthacrylate de méthoxy-poly(éthylène glycol 550), fourni par SARTOMER Chemicals;
- le M90G (méthacrylate de méthoxy-poly(éthylène glycol (9 unités de répétition)) et le M230G (méthacrylate de méthoxy-polyéthylène glycol (23 unités de répétitions)) disponibles chez Shin-Nakamura Chemicals;
- les méthacrylates de méthoxy-poly(éthylène glycol) de poids moléculaires moyens 300, 475 ou 1100, disponibles chez Sigma-Aldrich;
- l'acrylate de méthoxy-poly(éthylène glycol) de poids moléculaire moyen 426 disponible chez Sigma-Aldrich;
- les méthacrylates de méthoxy-poly(éthylène glycol) disponibles chez LAPORTE sous les dénominations commerciales : MPEG 350, MPEG 550, S10W, S20W.

Le monomère de formule (I), seul ou en mélange, est présent à raison de 10 à 80% en poids, par rapport au poids du polymère final, notamment de 20 à 70% en poids, de préférence de 30 à 60% en poids.

Le copolymère éthylénique selon l'invention comprend également un ou plusieurs monomères anioniques, ou l'un de leurs sels, choisis parmi l'anhydride maléique et les monomères de formule (II).
On entend par monomère anionique, un monomère qui peut être porteur d'une charge négative entre pH 4 et pH 12.

Les monomères peuvent donc être de formule : 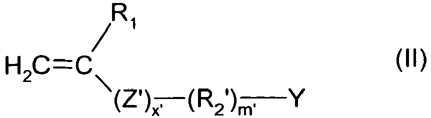 dans laquelle :
- R1 est un atome d'hydrogène ou un radical hydrocarboné, linéaire ou ramifié, de type CₚH₂ₚ₊₁, avec p étant un entier compris entre 1 et 12, inclus; Notamment, R1 peut représenter un radical méthyle, éthyle, propyle, butyle. De préférence, R1 représente l'hydrogène ou un radical méthyle.
- Z' est un groupement divalent choisi parmi -COO-, -OCO- ou -O-, -SO₂- -CO-O-CO- ou -CO- CH₂-CO-; de préférence, Z' est -COO-.
- x' est 0 ou 1, de préférence 1.
- R'2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;

Dans le radical R'2, le ou les hétéroatomes, quand ils sont présents, peuvent être intercalés dans la chaîne dudit radical R'2, ou bien ledit radical R'2 peut être substitué par un ou plusieurs groupes les comprenant tels que hydroxy.

Notamment R'2 peut être ou peut comprendre :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄- (ortho, méta ou para) éventuellement substitué, par un radical alkyle en C1-C12 comprenant éventuellement 1 à 25 hétéroatomes choisis parmi N, O, S, F, Si et/ou P; ou bien un radical benzylène-C₆H₄-CH₂- éventuellement substitué, par un radical alkyle en C1-C12 comprenant éventuellement 1 à 25 hétéroatomes choisis parmi O, N, S, F, Si et P;
- un radical de formule -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH-, -CH₂-CH₂-NH-CO-NH-, -CH₂-CHOH-, -CH₂-CH₂-CHOH-, -CH₂-CH₂-CH₂-O-; -CH₂-CH₂-CHR'-O- avec R' représentant un alkyle linéaire ou ramifié en C1-C22 comprenant éventuellement 1 à 12 hétéroatomes choisis parmi O, N, S, F, Si et P;
- leurs mélanges.
- m' est 0 ou 1;
- Y est un groupement choisi parmi -COOH, -SO₃H, -OSO₃H, -PO₃H₂ et -OPO₃H₂.

La neutralisation des groupes acides peut être effectuée par une base minérale ou organique comme indiquée plus haut telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la dimethylamino-2-propanol. On peut encore citer la lysine ou la 3-(diméthylamino)propylamine.

Il est entendu que selon l'état de l'art, les groupes SO₄H₂ et PO₄H₂ sont liés à R'2 par l'atome d'oxygène tandis que les groupes SO₃H et PO₃H sont liés à R'2 respectivement via les atomes de S et P.

Parmi les monomères anioniques préférés, on peut citer l'anhydride maléique, l'acide acrylique, l'acide méthacrylique, l'acide crotonique, l'acide itaconique, l'acide fumarique, l'acide maléique, l'acrylate de 2-carboxyéthyle
(CH2=CH-C(O)-O-(CH₂)₂-COOH); l'acide styrènesulfonique, l'acide vinylbenzoïque, l'acide vinylphosphorique, le (méth)acrylate de sulfopropyle, le (méth)acrylate de sulfatoéthyle et les sels de ceux-ci, notamment les sels d'ammonium.

Le monomère anionique, seul ou en mélange, est présent à raison de 20 à 90% en poids par rapport au poids du polymère final, notamment de 30 à 80% en poids, de préférence de 40 à 70% en poids.

Le copolymère éthylénique selon l'invention comprend également d'autres monomères que ceux mentionnés ci-dessus, choisis parmi les monomères non ioniques dits hydrophiles au sens de la présente invention.

Par monomère hydrophile, on entend les monomères ayant une valeur du logarithme du coefficient de partage apparent octanol-1/eau, aussi appelé log P, inférieure ou égale à 2, par exemple comprise entre -8 et 2, de préférence inférieure ou égale à 1,5, notamment inférieure ou égale à 1, et en particulier comprise entre - 7 et 1, voire entre 6 et 0.

Les valeurs de log P sont connues et sont déterminées selon un test standard qui détermine la concentration du monomère dans l'octanol et l'eau.

Les valeurs peuvent notamment être calculées à l'aide du logiciel ACD (Advanced Chemistry Development) Software solaris V4.67; elles peuvent également être obtenues à partir de Exploring QSAR : hydrophobic, electronic and steric constants (ACS professional reference book, 1995).

Il existe encore un site Internet qui fournit des valeurs estimées (adresse : www.srcinc.com).

Nous indiquons ci-après la valeur du log P de certains monomères usuels, déterminée à l'aide du logiciel ACD :

| | méthacrylate (* ou méthacrylamide) | Acrylate (* ou acrylamide) |
|---|---|---|
| Méthyl (méth)acrylate | 1.346 +/- 0.250 | 0.793 +/- 0.223 |
| Ethyl (méth)acrylate | 1.877 +/- 0.250 | 1.325 +/- 0.223 |
| Propyl (méth)acrylate | 2.408 +/- 0.250 | 1.856 +/- 0.223 |
| Isopropyl (méth)acrylate | 2.224 +/- 0.254 | 1.672 +/- 0.228 |
| Butyl (méth)acrylate | 2.940 +/- 0.250 | 2.387 +/- 0.223 |
| Isobutyl (méth)acrylate | 2.756 +/- 0.254 | 2.208+/-0.228 |
| terbutyl(méth)acrylate | 2.574 +/- 0.261 | 2.022 +/- 0.238 |
| cyclohexyle (méth)acrylate | 3.405 +/-0.252 | 2.853+/- 0.226 |
| octyl(méth)acrylate | 5.065 +/- 0.521 | 4.513 +/- 0.224 |
| lauryl(méth)acrylate | 7.190 +/- 0.251 | 6.638 +/- 0.224 |
| tridecyl(méth)acrylate | 7.712+/-0.251 | 7.170+/- 0.224 |
| cétyl (méth)acrylate | 9.316+/-0.251 | 8.764+/- 0.224 |
| palmityl(méth)acrylate | >9 | >9 |
| stéaryl (méth)acrylate | 10.379+/- 0.251 | 9.826+/-0.224 |
| behenyl (méth)acrylate | 11.952 +/- 0.225 | 12.504 ± 0.251 |
| oléyl (méth)acrylate | >9 | 9.308 ± 0.232 |
| Tetrahydrofurfuryl (méth)acrylate | 1,352 ± 0,283 | 0,800 ± 0,263 |
| 2-ethyl hexyl (méth)acrylate | 4,881 ± 0,254 | 4,329 ± 0,229 |
| 2-hydroxyethyl (méth)acrylate | 0,718 ± 0,277 | 0,166 ± 0,258 |
| éthoxyéthyle (méth)acrylate | 1,887 ± 0,293 | 1,335 ± 0,268 |
| Hydroxypropyl (méth)acrylate | | 0,383 ± 0,241 |
| N-isopropyle (méth)acrylamide * | 0,748 ± 0,276 | 0,195 ± 0,256 |
| N-octyl (méth)acrylamide * | 3,558 ± 0,273 | 3,036 ± 0,253 |
| N,N-diméthyl (méth)acrylamide* | 0,906 ± 0,553 | -0,168 ± 0,556 |
| N, N-dibutyl (méth)acrylamide* | 3,573 ± 0,570 | 3,021 ± 0,557 |
| | | |
| Acétate de vinyle | 0,730 ± 0,286 | |
| méthyl vinyl éther | 0,509 ± 0,286 | |
| éthyl vinyl éther | 1,040 ± 0,286 | |
| vinylcaprolactame | 1,499 ± 0,207 | |

| | méthacrylate (* ou méthacrylamide) | Acrylate (* ou acrylamide) |
|---|---|---|
| Vinylpyrrolidone | 0,370 ± 0,206 | |
| N-vinyl acétamide | 0 ± 0,231 | |

Les monomères hydrophiles additionnels sont choisis parmi ceux de formule (III), seuls ou en mélange, : 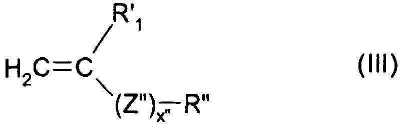 dans laquelle :
- R'₁ est l'hydrogène ou -CH₃;
- Z" est un groupement divalent choisi parmi -COO-, -CONH-, -CONCH₃-, -OCO-, -SO₂, -CO- O- CO-, -CO- CH₂-CO- ou- O- ; de préférence COO et CONH;
- x" est 0 ou 1;
- R" est un radical carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;

Dans le radical R", le ou les hétéroatomes, quand ils sont présents, peuvent être intercalés dans la chaîne dudit radical ou bien ledit radical peut être substitué par un ou plusieurs groupes les comprenant tels que hydroxy, ester, amide, uréthane ou urée.

Notamment R" peut être un radical méthyle, éthyle, propyle, isopropyle, n-butyle, isobutyle, tertiobutyle, phényle, benzyle, ou un radical de formule -CH₂-CH₂-CH₂OH, -CH₂-CH₂-OH, -CH₂-CH₂-CH₂OH ou furfuryle.

Les monomères non ioniques hydrophiles additionnels sont notamment choisis parmi les monomères ci-après : le méthacrylate de méthyle, l'acrylate de méthyle, le méthacrylate d'éthyle, l'acrylate d'éthyle, l'acrylate de propyle, l'acrylate d'isopropyle, le méthacrylate de tetrahydrofurfuryle, l'acrylate de tetrahydrofurfuryle, le méthacrylate de 2-hydroxyéthyle, l'acrylate de 2-hydroxyéthyle, le méthacrylate d'éthoxyéthyle, l'acrylate d'éthoxyéthyle, le N-isopropyl acrylamide, le N-isopropyl méthacrylamide, le N,N-diméthyl acrylamide, le N,N-diméthyl méthacrylamide, l'acétate de vinyle, le méthyl vinyl éther, l'éthyl vinyl éther, l'acrylate d'hydroxypropyle, la N-méthyl acrylamide, la N,N-diméthyl acrylamide.

Le monomère additionnel, seul ou en mélange, peut être présent en une quantité de 0,1 à 35% en poids, de préférence de 1 à 25% en poids, par exemple de 3 à 15% en poids, voire de 5 à 9,5% en poids, par rapport au poids total du polymère.

Le polymère final est de préférence un polymère comportant une charge globale négative, bien qu'il puisse comprendre d'éventuels motifs amines.

Les polymères selon l'invention peuvent être préparés selon les méthodes usuelles de polymérisation radicalaire classique, bien connues de l'homme du métier, et telles que décrites par exemple dans l'ouvrage "Chimie et physico-chimie des polymères" de Gnanou et al. (édition Dunod).

Notamment ces polymères peuvent être préparés par :
- polymérisation directe en solution dans l'eau avec pré-neutralisation ou non du motif anionique;
- polymérisation en émulsion dans l'eau avec pré-neutralisation ou non du motif anionique, avec utilisation d'un tensioactif;
- polymérisation dans un solvant organique, tel que l'éthanol ou la méthyléthylcétone, avec pré-neutralisation ou non du motif anionique, suivie d'une étape de mise en solution ou dispersion dans l'eau avec évaporation du solvant.

Ces polymérisations peuvent être effectuées en présence d'amorceur radicalaire notamment de type peroxyde (Trigonox 21 S: tert-Butyl peroxy-2-ethylhexanoate) ou azoïque (AIBN V50 : 2,2'-azo-bis(2-amidinopropane) dihydrochlorure), qui peut être présent à raison de 0,3 à 5% en poids par rapport au poids total des monomères.

Les polymères selon l'invention sont non réticulés. Ils se présentent sous forme de copolymères éthyléniques statistiques, de préférence filmogènes, d'un ou plusieurs monomères éthyléniques contenant des groupes PEG (les groupes PEG sont pendants le long du squelette) et d'un ou plusieurs monomères éthyléniques comportant des fonctions anioniques et, éventuellement d'un ou plusieurs autres comonomères éthyléniques hydrophiles non ioniques, monovalents.

Par polymère "éthylénique" , on entend un polymère obtenu par polymérisation de monomères comprenant une insaturation éthylénique.

Par polymère "filmogène", on entend un polymère apte à former à lui seul ou en présence d'un agent auxiliaire de filmification, un film continu et adhérent sur un support, notamment sur les matières kératiniques.

Ils présentent une masse moléculaire moyenne en poids (Mw) qui est de préférence comprise entre 500 et 5 000 000, notamment comprise entre 1 000 et 3 000 000 et plus préférentiellement comprise entre 2 000 et 2 000 000, voire 4 000 et 500 000, entre autre 7 000 et 250 000, encore mieux 8 000 et 100 000.

On détermine les masses molaires moyennes en poids (Mw) par chromatographie par perméation sur gel ou par diffusion de la lumière, selon l'accessibilité de la méthode (solubilité des polymères considérés).

Les polymères selon l'invention sont de préférence véhiculables en milieu aqueux, c'est-à-dire qu'ils sont de préférence hydrosolubles ou hydrodispersibles.

La mise en solution ou en dispersion dans l'eau peut être effectuée par solubilisation directe du polymère s'il est soluble, ou bien par neutralisation des motifs acides de façon à rendre le polymère soluble ou dispersible dans l'eau.

La mise en solution ou dispersion aqueuse peut également s'effectuer via une étape intermédiaire de solubilisation dans un solvant organique suivie de l'addition d'eau avant évaporation du solvant organique.

Par ailleurs, on a constaté que les polymères selon l'invention présentent avantageusement une viscosité dans l'eau adéquate avec les applications envisagées, qui peut être, par exemple comprise entre 1 et 1000 mPa.s, de préférence entre 1,5 et 750 mPa.s, et encore mieux entre 2 et 500 mPa.s.

La viscosité est mesurée à l'aide d'un viscosimètre BROOKFIELD, pour une solution à 15% en poids de polymère dans l'eau ou la méthyléthylcétone (solvant choisi en fonction de la solubilité du polymère et/ou de la méthode de polymérisation), à 25°C, avec un mobile de type aiguille (spindle) choisi parmi les modèles numéros 00 à 07 de Brookfield, de préférence le mobile no 1; pour un temps de mesure de 5 minutes, à une vitesse comprise entre 0,1 et 6 tours/minute, de préférence 6 tours/minute. La viscosité est mesurée après complète dissolution du polymère dans l'eau ou la méthyléthylcétone.

L'homme du métier pourra choisir le mobile sur la base de ses connaissances générales. La vitesse est également choisie de manière à pouvoir réaliser la mesure pour des composés liquides peu visqueux, dans une gamme de précision acceptable.

En outre, les polymères selon l'invention présentent de préférence une température de transition vitreuse (Tg) comprise entre -150°C et 20°C, notamment -120°C et 10°C, mieux entre -100°C et 0°C; la Tg est mesurée selon la méthode donnée avant les exemples.

Les polymères selon l'invention peuvent également présenter de préférence une température de fusion (Tf) comprise entre -100°C et 80°C, notamment entre -80°C et 50°C, mieux entre -70°C et 45°C, voire -10°C et 25°C.

En outre, les polymères selon l'invention présentent de préférence une reprise en eau comprise entre 3% et 150% en poids, de préférence entre 4% et 120% en poids, notamment entre 4,5% et 90% en poids, à 75% d'humidité relative (75%HR); la reprise en eau est mesurée selon la méthode donnée avant les exemples.

Ils peuvent également présenter une reprise en eau comprise entre 5% et 90% en poids, de préférence entre 7,5% et 75% en poids, notamment entre 15% et 60% en poids, à 85% d'humidité relative (85%HR).

Les polymères selon l'invention trouvent une application toute particulière dans le domaine de la cosmétique. Ils peuvent être présents dans la composition sous forme solubilisée, par exemple dans l'eau ou un solvant organique, ou bien sous forme de dispersion aqueuse ou organique.

Ils peuvent être utilisés dans les compositions cosmétiques ou pharmaceutiques à raison de 0,01 à 50% en poids de matière sèche, notamment 0,1 à 30% en poids, voire 1 à 25% en poids, encore mieux 3 à 20% en poids, par rapport au poids total de la composition.

Les compositions cosmétiques ou pharmaceutiques selon l'invention comprennent, outre lesdits polymères, un milieu physiologiquement acceptable, notamment cosmétiquement ou dermatologiquement acceptable, c'est-à-dire un milieu compatible avec les matières kératiniques telles que la peau du visage ou du corps, les cheveux, les cils, les sourcils et les ongles.

La composition peut ainsi comprendre, un milieu hydrophile comprenant de l'eau ou un mélange d'eau et de solvant(s) organique(s) hydrophile(s) comme les alcools et notamment les monoalcools, linéaires ou ramifiés en C1-C6, comme l'éthanol, le tert-butanol, le n-butanol, l'isopropanol ou le n-propanol, et les polyols comme la glycérine, la diglycérine, le propylène glycol, le sorbitol, le pentylène glycol, et les polyéthylène glycols, ou bien encore les éthers de glycols notamment en C₂ et des aldéhydes en C₂-C₄ hydrophiles.

L'eau ou le mélange d'eau et de solvants organiques hydrophiles peut être présent dans la composition selon l'invention en une teneur allant de 0,1% à 99% en poids, par rapport au poids total de la composition, et de préférence de 10% à 80% en poids.

La composition peut également comprendre une phase grasse, notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.

Comme corps gras liquides à température ambiante, appelés souvent huiles, utilisables dans l'invention, on peut citer : les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol; des alcools gras ayant de 12 à 26 atomes de carbone comme l'octyldodécanol, le 2-butyloctanol, le 2-hexyldécanol, le 2-undécylpentadécanol, l'alcool oléique; les huiles fluorées partiellement hydrocarbonées et/ou siliconées; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes; leurs mélanges.

Ces huiles peuvent être présentes en une teneur allant de 0,01 à 90%, et mieux de 0,1 à 85% en poids, par rapport au poids total de la composition.

La composition selon l'invention peut également comprendre un ou plusieurs solvants organiques, physiologiquement acceptables.

Ces solvants peuvent être généralement présents en une teneur allant de 0,1 à 90%, de préférence de 0,5 à 85%, de préférence encore de 10 à 80% en poids, par rapport au poids total de la composition, et mieux de 30 à 50 %.

On peut notamment citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylène glycol; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à 25°C tels que le diéthyléther, le diméthyléther ou le dichlorodiéthyléther; les alcanes liquides à 25°C tels que le décane, l'heptane, le dodécane, l'isododécane, le cyclohexane; les composés cycliques aromatiques liquides à 25°C tels que le toluène et le xylène ; les aldéhydes liquides à 25°C tels que le benzaldéhyde, l'acétaldéhyde et leurs mélanges.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METTLER.

Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,1 à 50% en poids de cires, par rapport au poids total de la composition et mieux de 1 à 30% en poids.

La composition selon l'invention peut en outre comprendre, dans une phase particulaire, des pigments et/ou des nacres et/ou des charges habituellement utilisés dans les compositions cosmétiques.

La composition peut également comprendre d'autres matières colorantes choisies parmi les colorants hydrosolubles ou les colorants liposolubles bien connus de l'homme du métier.

Par pigments, il faut comprendre des particules de toute forme, blanches ou colorées, minérales ou organiques, insolubles dans le milieu physiologique, destinées à colorer la composition.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Par nacres, il faut comprendre des particules de toute forme irisées, notamment produites par certains mollusques dans leur coquille ou bien synthétisées.

Les pigments peuvent être présents dans la composition à raison de 0,01 à 25% en poids de la composition finale, et de préférence à raison de 3 à 10% en poids. Ils peuvent être blancs ou colorés, minéraux ou organiques. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.

Les nacres peuvent être présentes dans la composition à raison de 0,01 à 20% en poids, de préférence à un taux de l'ordre de 3 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Parmi les colorants, liposolubles ou hydrosolubles, qui peuvent être présents dans la composition, seul ou en mélange, à raison de 0,001 à 15% en poids, de préférence 0,01 à 5% en poids et notamment de 0,1 à 2% en poids, par rapport au poids total de la composition, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène, le carmin de cochenille, les colorants halogéno-acides, azoïques, anthraquinoniques, le sulfate de cuivre ou de fer, le brun Soudan, le rouge Soudan et le rocou, ainsi que le jus de betterave et le carotène.

La composition selon l'invention peut comprendre en outre en outre une ou plusieurs charges, notamment en une teneur allant de 0,01% à 50% en poids, par rapport au poids total de la composition, de préférence allant de 0,02% à 30% en poids. Les charges peuvent être minérales ou organiques de toute forme, plaquettaires, sphériques ou oblongues. On peut citer le talc, le mica, la silice, le kaolin, les poudres de polyamide (Nylon®), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Coming) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition peut également comprendre avantageusement au moins un agent tensioactif qui est généralement présent en une quantité comprise entre 0,01 % et 50% en poids, de préférence entre 0,1% et 40% et encore plus préférentiellement entre 0,5% et 30%, par rapport au poids total de la composition.

Cet agent tensioactif peut être choisi parmi les agents tensioactifs anioniques, amphotères, non ioniques, cationiques ou leurs mélanges.

Les tensioactifs convenant à la mise en oeuvre de la présente invention sont notamment, seuls ou en mélange, :
- les tensioactifs anioniques parmi lesquels on peut citer, seuls ou mélanges, les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffinesulfonates; les alkylsulfosuccinates, les alkyléthersulfosuccinates, les alkylamidesulfosuccinates; les alkylsulfosuccinamates; les alkylsulfoacétates ; les alkylétherphosphates; les acylsarcosinates; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle.

On peut également citer les sels d'acides gras tels que les sels des acides oléique, ricinoléique, palmitique, stéarique, les acides d'huile de coprah ou d'huile de coprah hydrogénée; les acyl-lactylates dont le radical acyle comporte 8 à 20 atomes de carbone; les acides d'alkyl D galactoside uroniques et leurs sels ainsi que les acides alkyl (C₆-C₂₄) éther carboxyliques polyoxyalkylénés, les acides alkyl (C₆-C₂₄) aryl éther carboxyliques polyoxyalkylénés, les acides alkyl (C₆-C₂₄) amido éther carboxyliques polyoxyalkylénés et leurs sels, en particulier ceux comportant de 2 à 50 groupements oxyde d'éthylène, et leurs mélanges. - les tensioactifs non ioniques parmi lesquels on peut citer, seuls ou mélanges, les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les copolymères d'oxyde d'éthylène et de propylène, les condensats d'oxyde d'éthylène et de propylène sur des alcools gras; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.
- les tensioactifs amphotères parmi lesquels on peut citer, seuls ou mélanges, les dérivés d'amines secondaires ou tertiaires aliphatiques, dans lesquels le radical aliphatique est une chaîne linéaire ou ramifiée comportant 8 à 22 atomes de carbone et comprenant au moins un groupe anionique hydrosolubilisant (par exemple carboxylate, sulfonate, sulfate, phosphate ou phosphonate); on peut citer encore les alkyl (C₈-C₂₀) bétaïnes, les sulfobétaïnes, les alkyl (C₈-C₂₀) amidoalkyl (C₁-C₆) bétaïnes telles que la cocoamidopropylbétaïne ou les alkyl (C₈-C₂₀) amidoalkyl (C₁-C₆) sulfobétaïnes.
- les tensioactifs cationiques parmi lesquels on peut citer, seuls ou mélanges,
   A) les sels d'ammonium quaternaires de la formule générale (XVI) suivante : 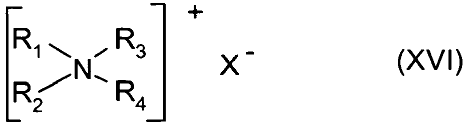 dans laquelle X est un anion choisi parmi les halogénures (chlorure, bromure ou iodure) ou alkyl (C₂-C₆) sulfates plus particulièrement méthylsulfate, des phosphates, des alkyl- ou -alkylarylsulfonates, des anions dérivés d'acide organique tel que l'acétate ou le lactate, et
      a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
         R4 désigne un radical alkyle, linéaire ou ramifié, comportant de 16 à 30 atomes de carbone. De préférence le tensioactif cationique est un sel (par exemple chlorure) de béhényl triméthyl ammonium.
      b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
         R3 et R4, identiques ou différents, désignent un radical alkyle, linéaire ou ramifié, comportant de 12 à 30 atomes de carbone, ledit radical comprenant au moins une fonction ester ou amide.
         R3 et R4 sont notamment choisis parmi les radicaux alkyl (C₁₂-C₂₂)amido alkyle (C₂-C₆), alkyl (C₁₂-C₂₂)acétate ;
         De préférence le tensioactif cationique est un sel (par exemple chlorure) de stéaramidopropyl diméthyl (myristylacétate) ammonium.
   B) - les sels d'ammonium quaternaire de l'imidazolinium, comme par exemple celui de formule (XVII) suivante : 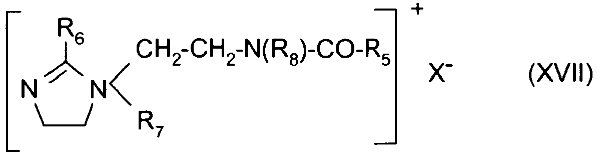 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
   C) - les sels de diammonium quaternaire de formule (XVIII) : 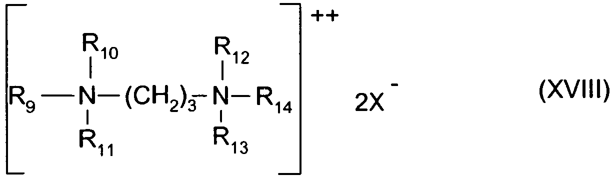 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
   D) - les sels d'ammonium quaternaire comprenant au moins une fonction ester de formule (XIX) suivante : 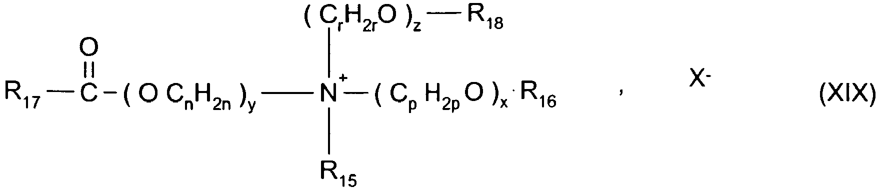
   dans laquelle :
   - R15 est choisi parmi les radicaux alkyles en C₁-C₆ et les radicaux hydroxyalkyles ou dihydroxyalkyles en C1-C6 ;
   - R16 est choisi parmi :
      - le radical 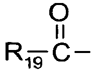
      - les radicaux R20 hydrocarbonés en C1-C22 linéaires ou ramifiés, saturés ou insaturés,
      - l'atome d'hydrogène,
   - R18 est choisi parmi :
      - le radical 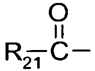
      - les radicaux R22 hydrocarbonés en C1-C6 linéaires ou ramifiés, saturés ou insaturés,
      - l'atome d'hydrogène,
   - R17, R19 et R21, identiques ou différents, sont choisis parmi les radicaux hydrocarbonés en C7-C22, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X- est un anion simple ou complexe, organique ou inorganique ;
sous réserve que la somme x + y + z vaut de 1 à 15, que lorsque x vaut 0 alors R16 désigne R20 et que lorsque z vaut 0 alors R18 désigne R22.

La composition selon l'invention peut également comprendre des ingrédients couramment utilisés en cosmétique, tels que les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Dans un mode de réalisation préféré, la composition selon l'invention et comprend au moins un conservateur; on entend par conservateur, tout additif technologique, naturel ou artificiel, qui a pour but de préserver la composition cosmétique de toute altération physico-chimique et microbienne. On peut ainsi citer par exemple :
- les conservateurs antimicrobiens : parabènes ou esters de l'acide 4-hydroxybenzoïque, acide sorbique, 2-phénoxyéthanol, formol, Triclosan, ammonium quaternaires,
- les conservateurs antioxydants : Tocophérol ou vitamine E, huiles essentielles, palmitate d'ascorbyle, BHT, BHA, gallates.

La composition selon l'invention peut se présenter sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray. Cette composition peut avoir l'aspect d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres et des cheveux, d'un produit solaire ou autobronzant, voire d'un produit capillaire. Elle trouve notamment une application particulièrement intéressante dans le domaine capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Dans un mode de réalisation préféré, les compositions conformes à l'invention peuvent être utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention peuvent être des compositions détergentes telles que des shampooings, des gels-douche et des bains moussants. Dans ce mode de réalisation de l'invention, les compositions comprennent au moins une base lavante, généralement aqueuse.

L'invention a aussi pour objet un procédé cosmétique de traitement, notamment de maquillage ou de soin, des matières kératiniques, notamment de la peau du corps ou du visage, des ongles, des cheveux, des poils et/ou des cils, comprenant l'application sur lesdites matières d'une composition cosmétique telle que définie précédemment.

L'application peut éventuellement être suivie d'un rinçage à l'eau. Ainsi, ce procédé selon l'invention permet le maintien de la coiffure, le traitement, le soin ou le lavage ou le démaquillage de la peau, des cheveux ou de toute autre matière kératinique.

Dans un autre mode de réalisation préféré, les compositions de l'invention peuvent se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle contient avantageusement au moins un tensioactif cationique, par exemple en une concentration généralement comprise entre 0,1 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

L'invention est illustrée plus en détail dans les exemples suivants.

### Mesure de la Tg

Un film est réalisé à partir d'une solution aqueuse à 6% en poids de polymère et séché 48 heures dans une atmosphère contrôlée à 50% d'humidité relative et 25°C. Les films ainsi obtenus ont une épaisseur comprise entre 10 et 20 µm. L'appareil de mesure est une DSC (TA instruments).

L'échantillon issu du film est déposé dans un creuset hermétique et est chauffé selon le protocole suivant :
- équilibre à température initiale Ti;
- chauffe 1 : augmentation de la température, à une vitesse de +10°C/min jusqu'à température finale :Tf (°C);
- isotherme durant 1 minute;
- diminution de la température à une vitesse de -10°C/min jusqu'à Ti (°C);
- chauffe 2 : augmentation de la température à une vitesse de +10°C/min jusqu'à Tf (°C);
- isotherme durant 1 minute.
avec Ti : Température initiale - 120°C
avec Tf Température finale + 120°C
Les Tg sont mesurées lors des étapes de chauffe 1 et 2.

### Mesure de la reprise en eau

On dépose environ 1 g de polymère sec dans une coupelle en aluminium de 4,5 cm de diamètre (0,01 m²) préalablement tarée T. On laisse sécher 48 heures à l'étuve à 60°C, sous pression réduite. On retire les coupelles et on les pèse immédiatement (moins d'une minute après leur sortie de l'étuve). On obtient P1.

Les coupelles sont ensuite placées dans une boite à gant ayant l'humidité relative considérée (75% HR ou 85% HR) et y sont laissées 6 heures. Elles sont ensuite pesées à nouveau immédiatement après leur sortie de la boite à gant. On obtient P2.

La reprise en eau est calculée de la manière suivante : [(P2-P1) x 100] / (P1-T)

### Exemple 1

Dans un réacteur (quadricol) surmonté de deux ampoules d'addition, d'un réfrigérant et muni d'une agitation mécanique, on introduit 50 ml de méthyléthylcétone (MEC), on porte à 80°C.

Parallèlement, on prépare une solution 1 comprenant 50 g de méthacrylate de polyéthylèneglycol (MPEG 550), 1 g de (Trigonox 21 S) et 75 g de MEC.

On prépare également une solution 2 comprenant 75 ml de MEC et 50 g d'acide acrylique.

Les solutions 1 et 2 sont coulées simultanément dans le réacteur quadricol en 30 minutes. Le mélange résultant est maintenu à 80°C ensuite pendant 5 heures. La solution jaune orangée obtenue est refroidie. On ajoute alors 690 ml de soude 1 N sous agitation, puis on procède à l'évaporation du solvant (MEC). On obtient 95 g de polymère.

### Exemple 2

De manière semblable à l'exemple 1, on fait réagir 50 g de méthacrylate de polyéthylèneglycol (MPEG 2000) et 50 g de méthacrylate d'ammonium sulfatoéthyle dans 200 ml d'eau en présence de 2,5 g de potassium persulfate. Ce mélange est chauffé 4 heures à 70°C puis refroidi à 25°C. On obtient une solution visqueuse orangée à 33% de matière sèche de polymère.

### Exemple 3

De manière semblable à l'exemple 1, on fait réagir 75 g de méthacrylate de polyéthylèneglycol (MPEG 2000) et 25 g d'acrylate de 3-sulfopropyle, sel de potassium dans 200 ml d'eau en présence de 2,5 g de potassium persulfate. Ce mélange est chauffé 4 heures à 70°C puis refroidi à 25°C. On obtient une solution jaune pâle à 30% de matière sèche de polymère.

### Exemple 4

De manière semblable à l'exemple 1, on fait réagir 75 g de méthacrylate de polyéthylèneglycol (MPEG 2000) et 25 g de méthacrylate d'ammonium sulfatoéthyle dans 200 ml d'eau en présence de 2,5 g de potassium persulfate. Ce mélange est chauffé 4 heures à 70°C puis refroidi à 25°C. On obtient une solution jaune pâle à 33% de matière sèche de polymère.

### Exemple 5

On mesure la reprise en eau, à HR 75%, et la viscosité des polymères ci-dessus préparés (viscosimètre BROOKFIELD, vitesse 6 tr/min, solution à 15% en poids de polymère dans l'eau, à 25°C, avec un mobile de type aiguille (spindle) n°1 de Brookfield, temps de mesure : 5 minutes).

Les résultats sont donnés dans le tableau ci-après:

| | Composition du polymère (% en poids) | Solubilité * | Reprise en eau | Viscosité à 15% |
|---|---|---|---|---|
| Exemple 1 | MPEG 550 50% | oui | 33% | 4 mPa.s |
| | Acide acrylique 50% | | | |
| | neutralisé NaOH 100% | | | |
| Exemple 2 | MPEG 550 50% | oui | 73% | 333 mPa.s |
| | méthacrylate de sulfatoéthyle, sel d'ammonium 50% | | | |
| Exemple 3 | MPEG 2000 75% | oui | 80% | 21 mPa.s |
| | Acrylate de 3-sulfopropyle, sel de potassium 25% | | | |
| Exemple 4 | MPEG 2000 75% | oui | 90% | 12,1 mPa.s |
| | méthacrylate de sulfatoéthyle, sel d'ammonium 25% | | | |
| *"oui" signifie que le polymère est soluble dans l'eau au moins jusqu'à 50% en poids. | | | | |

### Exemple 6

On prépare une composition de produit de coiffage comprenant les constituants suivants (% en poids):
- 6% de polymère décrit dans l'exemple 4 (à 33% de matière sèche)
- conservateur qs
- qsp 100% eau

La composition de produit de coiffage obtenue apporte un bon effet coiffant avec une élimination au shampooing aisée.

### Exemple 7

On prépare une composition de shampoing comprenant les constituants suivants (% en poids):
- 7,5% de lauryl éther sulfate
- 2,5% de tensioactif amphotère coco-bétaïne (Dehyton AB30 de Cognis)
- 5% de tensioactif coco-polyglucoside (Plantacare 818 UP de Cognis)
- 3% de polymère décrit dans l'exemple 2 (à 33% de matière sèche)
- qsp 100% eau

On constate en milieux humide et sec de bonnes propriétés de démêlage, et un coiffage amélioré.

## Revendications

1. Copolymère éthylénique comprenant, en % en poids par rapport au poids total du polymère :
- a) 10-80% en poids d'un ou plusieurs monomères de formule (I) : 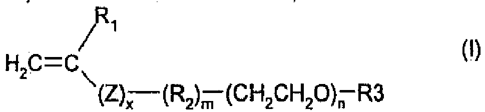 dans laquelle :
- R1 est un atome d'hydrogène ou un radical méthyle;
- Z est un groupement divalent -COO-;
- x est 1;
- m est 0
- n est un entier compris entre 3 et 300 inclus;
- R3 est un atome d'hydrogène ou un radical méthyle;
- b) 20-90% en poids d'un ou plusieurs monomères anioniques, ou l'un de leurs sels, choisis parmi l'anhydride maléique et les monomères de formule (II) : 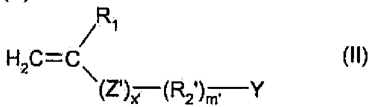 dans laquelle :
- R1 est un atome d'hydrogène ou un radical hydrocarboné, linéaire ou ramifié, de type CpH₂ₚ₊₁, avec p étant un entier compris entre 1 et 12, inclus;
- Z' est un groupement divalent choisi parmi -COO-, -OCO- ou -O-, -SO₂- -CO-O-CO- ou -CO-CH₂-CO-;
- X' est 0 ou 1;
- R'2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m'est 0 ou 1;
- Y est un groupement choisi parmi -COOH, -SO₃H, -OSO₃H, -PO₃H₂ et - OPO₃H2;
- c) 0,1 à 35% en poids d'au moins un monomère hydrophile non ionique additionnel choisi parmi ceux de formule (III), seuls ou en mélange, : 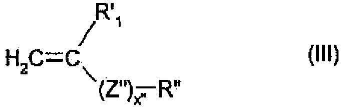 dans laquelle :
- R'₁ est l'hydrogène ou -CH₃;
- Z" est un groupement divalent choisi parmi -COO-, -CONH-, -CONCH₃-, - OCO-, - SO₂, -CO-O-CO-, -CO-CH₂-CO- ou -O- ;
- X" est 0 ou 1;
- R" est un radical carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P.

2. Polymère selon la revendication précédente, dans lequel, dans la formule (I), n est compris entre 5 et 200 inclus, et encore mieux entre 7 et 100 inclus, voire entre 9 et 50 inclus.

3. Polymère selon l'une des revendications précédentes, dans lequel le monomère de formule (I), seul ou en mélange, est présent à raison de 20 à 70% en poids, par rapport au poids du polymère final, de préférence de 30 à 60% en poids.

4. Polymère selon l'une des revendications précédentes, dans lequel dans la formule (II), Z' est COO.

5. Polymère selon l'une des revendications précédentes, dans lequel dans la formule (II), le radical R'2 est un radical choisi parmi :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 5 hétéroatomes choisis parmi N, O, S, F, Si et/ou P; ou bien un radical benzylène -C₆H₄-CH₂- éventuellement substitué, par un radical alkyle en C1-C12 comprenant éventuellement 1 à 5 hétéroatomes choisis parmi O, N, S, F, Si et P;
- un radical de formule -CH₂-O-CO-O-, CH₂-CH₂-O-CO-O-, -CH₂-CO-O-, -CH₂-CH₂-CO-O-, -CH₂-O-CO-NH- -CH₂-CH₂-O-CO-NH-; -CH₂-NH-CO-NH-, -CH₂-CH₂-NH-CO-NH-, -CH₂-CHOH-, -CH₂-CH₂-CHOH-, -CH₂-CH₂-CH₂-O-; -CH₂-CH₂-CHR^{'}-O- avec R' représentant un alkyle linéaire ou ramifié en C1-C22 comprenant éventuellement 1 à 12 hétéroatomes choisis parmi O, N, S, F, Si et P;
- ou un mélange de ces radicaux.

6. Polymère selon l'une des revendications précédentes, dans lequel les monomères anioniques sont choisis parmi l'anhydride maléique, l'acide acrylique, l'acide méthacrylique, l'acide crotonique, l'acide itaconique, l'acide fumarique, l'acide maléique, l'acrylate de 2-carboxyéthyle; l'acide styrènesulfonique, l'acide vinylbenzoïque, l'acide vinylphosphorique, le (méth)acrylate de sulfopropyle, le (méth)acrylate de sulfatoéthyle et les sels de ceux-ci, notamment les sels d'ammonium.

7. Polymère selon l'une des revendications précédentes, dans lequel le monomère anionique est présent à raison de 30 à 80% en poids par rapport au poids du polymère final, de préférence de 40 à 70% en poids.

8. Polymère selon l'une des revendications précédentes, dans lequel le monomère hydrophile non ionique est choisi parmi le méthacrylate de méthyle, l'acrylate de méthyle, le méthacrylate d'éthyle, l'acrylate d'éthyle, l'acrylate de propyle, l'acrylate d'isopropyle, le méthacrylate de tetrahydrofurfuryle, l'acrylate de tetrahydrofurfuryle, le méthacrylate de 2-hydroxyéthyle, l'acrylate de 2-hydroxyéthyle, le méthacrylate d'éthoxyéthyle, l'acrylate d'éthoxyéthyle, le N-isopropyl acrylamide, le N-isopropyl méthacrylamide, le N,N-diméthyl acrylamide, le N,N-diméthyl méthacrylamide, l'acétate de vinyle, le méthyl vinyl éther, l'éthyl vinyl éther, l'acrylate d'hydroxypropyle.

9. Polymère selon l'une des revendications précédentes, dans lequel le monomère hydrophile non ionique additionnel, seul ou en mélange, est présent en une quantité de 1 à 25% en poids, notamment 3 à 15% en poids, voire de 5 à 9,5% en poids, par rapport au poids total du polymère.

10. Polymère selon l'une des revendications précédentes, **caractérisée par le fait qu'**il est véhiculable en milieu aqueux, de préférence hydrosoluble ou hydrodispersible.

11. Composition cosmétique ou pharmaceutique comprenant dans un milieu physiologiquement acceptable, notamment cosmétiquement ou pharmaceutiquement acceptable, au moins un polymère tel que défini à l'une des revendications 1 à 10.

12. Composition selon la revendication 11, dans laquelle le polymère est présent à raison de 0,01 à 50% en poids de matière sèche, notamment de 0,1 à 30% en poids, voire de 1 à 25% en poids, encore mieux de 3 à 20% en poids, par rapport au poids total de la composition.

13. Composition selon l'une des revendications 11 à 12, dans laquelle le milieu physiologiquement acceptable comprend au moins un constituant choisi parmi l'eau ; les solvants organiques hydrophiles comme les alcools, notamment les monoalcools, linéaires ou ramifiés en C₁-C₆ et les polyols et les éthers de glycols notamment en C₂ et des aldéhydes en C₂-C₄ hydrophiles ; les cires, les corps gras pâteux, les gommes et leurs mélanges, d'origine animale, végétale, minérale ou synthétique ; des solvants organiques lipophiles ; des huiles d'origine animale, végétale, minérale ou synthétique ; les esters et les éthers de synthèse ; les esters du pentaérythritol; des alcools gras ayant de 12 à 26 atomes de carbone ; les huiles fluorées partiellement hydrocarbonées et/ou siliconées; les huiles siliconées volatiles ou non, linéaires ou cycliques, liquides ou pâteux à température ambiante; des pigments, des nacres, des charges ; les colorants hydrosolubles, les colorants liposolubles ; les polymères ; les agents auxiliaires de filmification ; les tensioactifs ; les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les antioxydants, les agents anti-chutes des cheveux, les agents anti-pelliculaires, les agents propulseurs, les céramides ; leurs mélanges.

14. Composition selon l'une des revendications 11 à 13, se présentant sous la forme d'un produit de soin et/ou de maquillage de la peau du corps ou du visage, des lèvres et des cheveux, d'un produit solaire ou autobronzant, d'un produit capillaire.

15. Composition selon l'une des revendications 11 à 14, se présentant sous la forme d'une composition capillaire, notamment pour le maintien de la coiffure ou la mise en forme des cheveux, par exemple sous forme de shampooings, de gels, de lotions de mise en plis, de lotions pour le brushing, de compositions de fixation et de coiffage telles que les laques ou spray ; d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

16. Procédé cosmétique de traitement des matières kératiniques telles que la peau du corps ou du visage, des ongles, des poils, des cheveux et/ou des cils, **caractérisé en ce qu'**il consiste à appliquer sur les matières kératiniques une composition cosmétique telle que définie dans l'une des revendications 11 à 15.

## Patentansprüche

1. Ethylenisches Copolymer, umfassend in Gew.-%, bezogen auf das Gesamtgewicht des Polymers:
- a) 10-80 Gew.-% eines oder mehrerer Monomere der Formel (I): 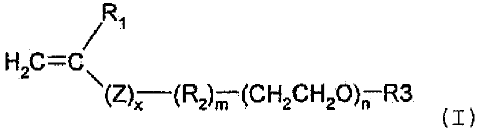 worin:
- R1 für ein Wasserstoffatom oder einen Methylrest steht;
- Z für eine zweiwertige Gruppe -COO- steht;
- x für 1 steht;
- m für 0 steht;
- n für eine ganze zahl zwischen 3 und 300 einschließlich steht;
- R3 für ein Wasserstoffatom oder einen Methylrest steht;
- b) 20-90 Gew.-% eines oder mehrerer anionischer Monomere oder eines Salzes davon, ausgewählt unter Maleinsäureanhydrid und den Monomeren der Formel (II) : 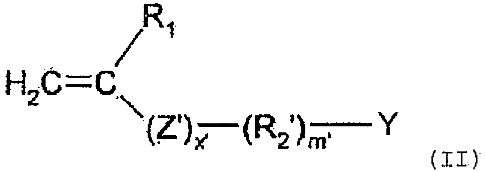 worin:
- R1 für ein Wasserstoffatom oder einen linearen oder verzweigten auf Kohlenwasserstoff basierenden Rest vom Typ CₚH₂ₚ₊₁, wobei p eine ganze zahl zwischen 1 und 12 einschließlich bedeutet, steht;
- Z' für eine unter -COO-, -OCO- oder -O-, -SO₂- - CO-O-CO- oder -CO-CH₂-CO- ausgewählte zweiwertige Gruppe steht;
- x' für 0 oder 1 steht;
- R'2 für einen gesättigten oder ungesättigten, gegebenenfalls aromatischen, linearen, verzweigten oder cyclischen auf Kohlenstoff basierenden zweiwertigen Rest mit 1 bis 30 Kohlenstoffatomen, der 1 bis 18 unter O, N, S, F, Si und P ausgewählte Heteroatome umfassen kann, steht;
- m' für 0 oder 1 steht;
- Y für eine unter -COOH, -SO₃H, -OSO₃H, -PO₃H₂ und -OPO₃H₂ ausgewählte Gruppe steht;
- c) 0,1% bis 35 Gew.-% mindestens eines zusätzlichen nichtionischen hydrophilen Monomers, das unter denjenigen der Formel (III): 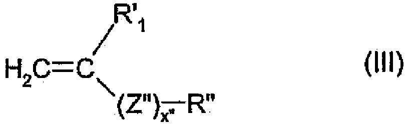 worin:
- R'₁ für Wasserstoff oder -CH₃ steht;
- Z" für eine unter -COO-, -CONH-, -CONCH₃-,
- OCO-, -SO₂ -CO-O-CO-, -CO-CH₂-CO- oder -O-ausgewählte zweiwertige Gruppe steht;
- x" für 0 oder 1 steht;
- R" für einen gesättigten oder ungesättigten, gegebenenfalls aromatischen, linearen, verzweigten oder cyclischen auf Kohlenstoff basierenden Rest mit 1 bis 30 Kohlenstoffatomen, der 1 bis 18 unter O, N, S, F, Si und P ausgewählte Heteroatome umfassen kann, steht;
allein oder als Mischung ausgewählt ist.

2. Polymer nach dem der vorhergehenden Anspruch, wobei in der Formel (I) n zwischen 5 und 200 einschließlich und noch besser zwischen 7 und 100 einschließlich oder sogar zwischen 9 und 50 einschließlich liegt.

3. Polymer nach einem der vorhergehenden Ansprüche, wobei das Monomer der Formel (I) allein oder als Mischung in einem Anteil von 20 bis 70 Gew.-%, bezogen auf das Gewicht des letztendlichen Polymers, vorzugsweise 30 bis 60 Gew.-%, vorliegt.

4. Polymer nach einem der vorhergehenden Ansprüche, wobei in der Formel (II) Z' für COO steht.

5. Polymer nach einem der vorhergehenden Ansprüche, wobei in der Formel (II) der Rest R'2 ein Rest ist, der unter:
- einem Alkylenrest wie Methylen, Ethylen, Propylen, n-Butylen, Isobutylen, tert.-Butylen, n-Hexylen, n-Octylen, n-Dodecylen, n-Octadecylen, n-Tetradecylen oder n-Docosanylen;
- einem Phenylenrest -C₆H₄- (ortho, meta oder para), der gegebenenfalls durch einen C1-C12-Alkylrest, der gegebenenfalls 1 bis 5 unter N, O, S, F, Si und/oder P ausgewählte Heteroatome umfasst, substituiert ist, oder einem Benzylenrest -C₆H₄-CH₂-, der gegebenenfalls durch einen C1-C12-Alkylrest, der gegebenenfalls 1 bis 5 unter O, N, S, F, Si und P ausgewählte Heteroatome umfasst, substituiert ist;
- einem Rest der Formel -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH-, -CH₂-CH₂-NH-CO-NH-, -CH₂-CHOH-, -CH₂-CH₂-CHOH-, -CH₂-CH₂-CH₂-O; -CH₂-CH₂-CHR'-O-, wobei R' für einen linearen oder verzweigten C1-C22-Alkylrest, der gegebenenfalls 1 bis 12 unter O, N, S, F, Si und P ausgewählte Heteroatome umfasst, steht;
- oder einer Mischung dieser Reste ausgewählt ist.

6. Polymer nach einem der vorhergehenden Ansprüche, wobei die anionischen Monomere unter Maleinsäureanhydrid, Acrylsäure, Methacrylsäure, 2-Carboxyethylacrylat; Styrolsulfonsäure, Vinylbenzoesäure, Vinylphosphorsäure, Sulfopropyl(meth)acrylat, Sulfatoethyl(meth)acrylat und Salzen davon, insbesondere Ammoniumsalzen ausgewählt sind.

7. Polymer nach einem der vorhergehenden Ansprüche, wobei das anionische Monomer in einem Anteil von 30 bis 80 Gew.-%, bezogen auf das Gewicht des letztendlichen Polymers, vorzugsweise 40 bis 70 Gew.-%, vorliegt.

8. Polymer nach einem der vorhergehenden Ansprüche, wobei das nichtionische hydrophile Monomer unter Methylmethacrylat, Methylacrylat, Ethylmethacrylat, Ethylacrylat, Propylacrylat, Isopropylacrylat, Tetrahydrofurfurylmethacrylat, Tetrahydrofurfurylacrylat, 2-Hydroxyethylmethacrylat, 2-Hydroxyethylacrylat, Ethoxyethylmethacrylat, Ethoxyethylacrylat, N-Methyläcrylamid, N-Isopropylacrylamid, N-Isopropylmethacrylamid, N,N-Dimethylacrylamid, N,N-Dimethylmethacrylamid, Vinylacetat, Methylvinylether, Ethylvinylether, Hydroxypropylacrylat ausgewählt ist.

9. Polymer nach einem der vorhergehenden Ansprüche, wobei das zusätzliche nichtionische hydrophile Monomer allein oder als Mischung in einer Menge von 1 bis 25 Gew.-%, insbesondere 3 bis 15 Gew.-% oder sogar 5 bis 9,5 Gew.-%, bezogen auf das Gesamtgewicht des Polymers, vorliegt.

10. Polymer nach einem der vorhergehenden Ansprüche, **dadurch gekennzeichnet, dass** es in wässriges Medium überführbar und vorzugsweise wasserlöslich oder wasserdispergierbar ist.

11. Kosmetische oder pharmazeutische Zusammensetzung, die in einem physiologisch unbedenklichen und insbesondere kosmetisch oder pharmazeutisch unbedenklichen Medium mindestens ein Polymer gemäß einem der Ansprüche 1 bis 10 umfasst.

12. Zusammensetzung nach Anspruch 11, in der das Polymer in einem Anteil von 0,01 bis 50 Gew.-% Trockensubstanz, insbesondere 0,1 bis 30 Gew.-% oder sogar 1 bis 25 Gew.-% oder noch besser 3 bis 20 Gew.-%, bezogen auf das Gesamtgewicht der Zusammensetzung, vorliegt.

13. Zusammensetzung nach einem der Ansprüche 11 bis 12, in der das physiologisch unbedenkliche Medium mindestens einen unter Wasser; hydrophilen organischen Lösungsmitteln wie Alkoholen, insbesondere linearen oder verzweigten C1-C6-Monoalkoholen und Polyolen und Glykolethern, insbesondere C₂-Glykolethern, und hydrophilen C₂-C₄-Aldehyden; Wachsen, pastösen Fettkörpern, Gummen und Mischungen davon tierischen, pflanzlichen, mineralischen oder synthetischen Ursprungs; organischen lipophilen Lösungsmitteln; Ölen tierischen, pflanzlichen, mineralischen oder synthetischen Ursprungs; synthetischen Estern und Ethern; Pentaerythritestern; Fettalkoholen mit 12 bis 26 Kohlenstoffatomen; teilfluorierten Kohlenwasserstoff- und/oder Silikonölen; flüchtigen oder nichtflüchtigen, linearen oder cyclischen, bei Raumtemperatur flüssigen oder pastösen Silikonölen; Pigmenten, Perlmutt, Füllstoffen; wasserlöslichen Farbmitteln; fettlöslichen Farbmitteln; Polymeren; Filmbildungshilfsmitteln; Tensiden; Vitaminen, Parfümen, Perlglanzmitteln, Verdickungsmitteln, Gelierungsmitteln, Spurenelementen, Glättungsmitteln, Sequestriermitteln, Parfümen, Alkalinisierungs- oder Ansäuerungsmitteln, Konservierungsmitteln, Sonnenschutzmitteln, Antioxidantien, Mitteln gegen Haarausfall, Antischuppenmitteln, Treibmitteln, Ceramiden und Mischungen davon ausgewählten Bestandteil umfasst.

14. Zusammensetzung nach einem der Ansprüche 11 bis 13, die in Form eines Produkts zur Pflege und/oder zum Make-up der Körper- oder Gesichtshaut, der Lippen und der Haare, eines Sonnenschutz- oder Selbstbräunungsprodukts oder eines Haarprodukts vorliegt.

15. Zusammensetzung nach einem der Ansprüche 11 bis 14, die in Form eines Haarprodukts, insbesondere zur Festigung der Frisur oder zur Haargestaltung, beispielsweise in Form von Shampoos, Gelen, Lotionen für die Wasserwelle, Lotionen für die Fönwelle, Zusammensetzungen zur Fixierung der Frisur wie Lacken oder Sprays; Wash-out- oder Leave-in-Haarpflegespülungen, Dauerwellen-, Entkrausungs-, Färbe- oder Bleichzusammensetzungen oder auch in Form von Wash-out-Zusammensetzungen zur Anwendung vor oder nach einer Färbung, Bleichung, Dauerwelle oder Entkrausung oder auch zwischen zwei Schritten einer Dauerwelle oder Entkrausung, vorliegt.

16. Verfahren zur kosmetischen Behandlung von Keratinmaterialien wie der Körper- oder Gesichtshaut, der Nägel, der Körperbehaarung, der Haare und/oder der Wimpern, **dadurch gekennzeichnet, dass** es daraus besteht, dass man auf die Keratinmaterialien eine kosmetische Zusammensetzung gemäß einem der Ansprüche 11 bis 15 aufbringt.

## Claims

1. Ethylenic copolymer comprising, as % by weight with respect to the total weight of the polymer:
- a) 10-80% by weight of one or more monomers of formula (I): 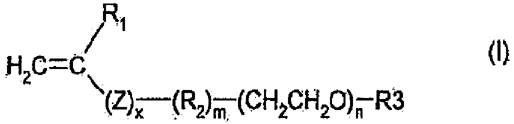 in which:
- R₁ is a hydrogen atom or a methyl radical;
- Z is a divalent-COO-group;
- x is 1;
- m is 0;
- n is an integer between 3 and 300 inclusive;
- R₃ is a hydrogen atom or a methyl radical;
- b) 20-90% by weight of one or more anionic monomers, of one of their salts, chosen from maleic anhydride and monomers of formula (II) : 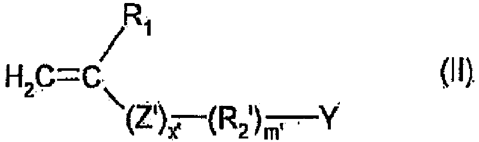
in which:
- R₁ is a hydrogen atom or a linear or branched hydrocarbon radical of CₚH₂ₚ₊₁ type with p being an integer between 1 and 12 inclusive;
- Z' is a divalent group chosen from -COO-, -OCO- or -O-, -SO₂- -CO-O-CO- or -CO-CH₂-CO-;
- x' is 0 or 1;
- R'₂ is a linear, branched or cyclic, saturated or unsaturated, optionally aromatic, divalent carbon-based radical with 1 to 30 carbon atoms which can comprise 1 to 18 heteroatoms chosen from O, N, S, F, Si and P;
- m' is 0 or 1;
- Y is a group chosen from -COOH, -SO₃H, -OSO₃H, -PO₃H₂ and -OPO₃H₂;
- c) 0.1 to 35% by weight of at least one additional nonionic hydrophilic monomer chosen from those of formula (III), alone or as a mixture: 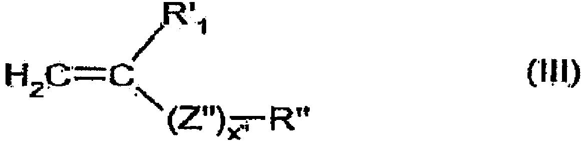 in which:
- R'₁ is hydrogen or -CH₃;
- Z" is a divalent group chosen from -COO-, -CONH-, -CONCH₃-, -OCO-, -SO₂, -CO-O-CO-, -CO-CH₂-CO- or -O-;
- x" is 0 or 1;
- R" is a linear, branched or cyclic, saturated or unsaturated, optionally aromatic, carbon-based radical with 1 to 30 carbon atoms which can comprise 1 to 18 heteroatoms chosen from O, N, S, F, Si and P.

2. Polymer according to the preceding claim, in which, in the formula (I), n is between 5 and 200 inclusive and better still between 7 and 100 inclusive, and indeed even between 9 and 50 inclusive.

3. Polymer according to either of the preceding claims, in which the monomer of formula (I), alone or as a mixture, is present in a proportion of 20 to 70% by weight, with respect to the weight of the final polymer, preferably of 30 to 60% by weight.

4. Polymer according to one of the preceding claims, in which, in the formula (II), Z' is COO.

5. Polymer according to one of the preceding claims, in which, in the formula (II), the radical R'₂ is a radical chosen from:
- an alkylene radical, such as methylene, ethylene, propylene, n-butylene, isobutylene, tert-butylene, n-hexylene, n-octylene, n-dodecylene, n-octadecylene, n-tetradecylene or n-docosanylene;
- a phenylene radical -C₆H₄- (ortho, meta or para) optionally substituted by a C₁-C₁₂ alkyl radical optionally comprising 1 to 5 heteratoms chosen from N, O, S, F, Si and/or P; or else a benzylene radical -C₆H₄-CH₂- optionally substituted by a C₁-C₁₂ alkyl radical optionally comprising 1 to 5 heteroatoms chosen from O, N, S, F, Si and P;
- a radical of formula -CH₂-O-CO-O-, CH₂-CH₂-O-CO-O-, -CH₂-CO-O-, -CH₂-CH₂-O-CO-O-,
-CH₂-O-CO-NH-, -CH₂-CH₂-O-CO-NH-, -CH₂-NH-CO-NH-, -CH₂-CH₂-NH-CO-NH-, -CH₂-CHOH-,
-CH₂-CH₂-CHOH-, -CH-CH₂-CH₂-O- or -CH₂-CH₂-CHR'-O-with R' representing a linear or branched C₁-C₂₂ alkyl optionally comprising 1 to 12 heteroatoms chosen from O, N, S, F, Si and P;
- or a mixture of these radicals.

6. Polymer according to one of the preceding claims, in which the anionic monomers are chosen from maleic anhydride, acrylic acid, methacrylic acid, 2-carboxyethyl acrylate, styrenesulfonic acid, vinylbenzoic acid, vinylphosphoric acid, sulfopropyl (meth)acrylate, sulfatoethyl (meth)acrylate and the salts of these, in particular the ammonia salts.

7. Polymer according to one of the preceding claims, in which the anionic monomer is present in a proportion of 30% to 80% by weight, with respect to the weights of the final polymer, preferably of 40 to 70% by weight.

8. Polymer according to one of the preceding claims, in which the nonionic hydrophilic monomer is chosen from methyl methacrylate, methyl acrylate, ethyl methacrylate, ethyl acrylate, propyl acrylate, isopropyl acrylate, tetrahydrofurfuryl methacrylate, tetrahydrofurfuryl acrylate, 2-hydroxyethyl methacrylate, 2-hydroxyethyl acrylate, 2-hydroxyethyl acrylate, ethoxyethyl methacrylate, ethoxyethyl acrylate, N-methylacrylamide, N-isopropylacrylamide, N-isopropylmethacrylamide, N,N-dimethylacrylamide, N,N-dimethylmethacrylamide, vinyl acetate, methyl vinyl ether, ethyl vinyl ether or hydroxypropyl acrylate.

9. Polymer according to one of the preceding claims, in which the additional nonionic hydrophilic monomer, alone or as a mixture, is present in an amount of 1 to 25% by weight, in particular 3 to 15% by weight, indeed even of 5 to 9.5% by weight, with respect to the total weight of the polymer.

10. Polymer according to one of the preceding claims, **characterized in that** it can be conveyed in an aqueous medium and is preferably water-soluble or waterdispersible.

11. Cosmetic or pharmaceutical composition comprising, in a physiologically acceptable medium, in particular a cosmetically or pharmaceutically acceptable medium, at least one polymer as defined in one of Claims 1 to 10.

12. Composition according to Claim 11, in which the polymer is present in a proportion of 0.01 to 50% by weight on a dry basis, in particular of 0.1 to 30% by weight, indeed even of 1 to 25% by weight, better still of 3 to 20% by weight, with respect to the total weight of the composition.

13. Composition according to either of Claims 11 and 12, in which the physiologically acceptable medium comprises at least one constituent chosen from water; hydrophilic organic solvents, such as alcohols, in particular linear or branched C₁-C₆ monoalcohols, polyols, glycol ethers, in particular C₂ glycol ethers, and C₂-C₄ aldehydes which are hydrophilic; waxes, pasty fatty substances, gums and their mixtures of animal, vegetable, mineral or synthetic origin; lipophilic organic solvents; oils of animal, vegetable, mineral or synthetic origin; synthetic esters and ethers; pentaerythritol esters; fatty alcohols having from 12 to 26 carbon atoms; partially hydrocarboncomprising and/or silicone-comprising fluorinated oils; volatile or nonvolatile and linear or cyclic silicone oils which are liquid or pasty at ambient temperature; pigments, pearlescent agents or fillers; water-soluble dyes or fat-soluble dyes; polymers; additional agents which are able to form a film; surfactants; vitamins, fragrances, pearlescent agents, thickeners, gelling agents, trace elements, softening agents, sequestering agents, basifying or acidifying agents, preservatives, sunscreens, antioxidants, agents for combatting hair loss, antidandruff agents, propellants or ceramides; or their mixtures.

14. Composition according to one of Claims 11 to 13, which is provided in the form of a product for caring for and/or making up the skin of the body or face, the lips and the hair, of an sun-care or self-tanning product, of a hair product.

15. Composition according to one of Claims 11 to 14, which is provided in the form of a hair composition, in particular for the form retention of the hairstyle or the shaping of the hair, for example in the form of shampoos, gels, hair setting lotions, blow drying lotions, or fixing and styling compositions, such as lacquers or sprays; of rinse-out or leave-in conditioners, of perming, hair straightening, dyeing or bleaching compositions, or else in the form of rinse-out compositions, to be applied before or after a dyeing, a bleaching, a perming or a hair straightening or else between the two stages of a perming or of a hair straightening.

16. Cosmetic method for treating keratinous substances, such as the skin of the body or face, the nails, the hair, including body hair, and/or the eyelashes, **characterized in that** it consists in applying, to the keratinous substances, a cosmetic composition as defined in one of Claims 11 to 15.
